Europäisches Patentamt

European Patent Office

Office européen des brevets

(11) Publication number: **0 273 085**
A1

(12) **EUROPEAN PATENT APPLICATION**

(21) Application number: 86810614.7

(51) Int. Cl.⁴: **C12N 15/00**

(22) Date of filing: 29.12.86

(43) Date of publication of application:
06.07.88 Bulletin 88/27

(84) Designated Contracting States:
AT BE CH DE ES FR GB GR IT LI LU NL SE

(71) Applicant: **BATTELLE MEMORIAL INSTITUTE**
7 route de Drize
CH-1227 Carouge/Genève(CH)

(72) Inventor: **Myers, André**
19, Grand Rue
CH-1204 Genève(CH)

(74) Representative: **Dousse, Blasco et al**
7, route de Drize
CH-1227 Carouge/Genève(CH)

(54) **A method for internalizing nucleic acids into eukaryotic cells.**

(57) A piece of useful nucleic acid is linked to a cell homing factor, for instance a cell receptor such as that for the epidermal growth factor (EGF), and the resulting coupled system is used to transfect and/or transform suitably selected host cells. This technique provides internalization in high yields and constitutes a significant advance over classic transfection, e.g. with CA⁺⁺ ions.

FIG. I

# A METHOD FOR INTERNALIZING NUCLEIC ACIDS INTO EUKARYOTIC CELLS

## INTRODUCTION

The present invention concerns a method for cell transfection i.e. for introducing and internalizing foreign sequences of nucleic acids into suitable eukaryotic host cells. In the instance when these nucleic acids are incorporated in the cell nucleus and become part of its genome, the result is cell transformation.

Until now cell transfection (and/or transformation) has been carried out by several methods including, for instance, working in the presence of Ca++ ions which promote the preferential uptake by cells of Ca-precipitated nucleic acids (F.L. GRAHAM et al. 1973, Virology 52, 456-467). Other methods also include for instance the direct injection of genes into oocyte nuclei (E.G. DIAKUMAKAS, Methods In Cell Biology, 1973 Academic Press, Vol 7, 287-311) as well as the use of viral vectors (D.H. Hamer et al. 1979, Cell 18, 1299), or liposomes dispersions for carrying the genes into the cells (R. Fraley 1980, J. Biol. Chem. 255, 10431). Transfections can also be brought about with DEAE-Dextran (J.H. McCutchan, 1968, G. Natl. Cancer Inst. 41, 351) and by genetic transfer in high voltage electric fields (E. Neumann et al. 1982, Embo J. Vol. 1, No. 7, 841-845).

Although all the above techniques have merit, they are not universally applicable. For example, with the DEAE-Dextran method exogenous DNA sequences are not efficiently integrated, this being in contrast to the method using calcium salts in which internalization of the DNA into the host genome frequently occurs. Direct injection into cells is tedious and requires highly skilled operators. Using a virus as a vector offers high yield transfections, but problems may arise from the simultaneous introduction of undesirable and possibly dangerous viral factors.

There is therefore a need for improved transfection techniques providing high internalization yields under mild conditions and of a highly selective character, i.e. making it possible to selectively transfect and, in some cases, transform one particular species of cells in the presence of other cells of different kinds. This technique is especially attractive when the nucleic acids to be incorporated can genetically modify only specifically defined target cells, or, in the case of cancer therapy, internally promote the expression of a cytotoxic substance while leaving normal cells intact.

## BRIEF DESCRIPTION OF THE INVENTION

The method disclosed in annexed Claim 1 is a very significant step toward the aforementioned desirable objectives. Briefly stated, a selected piece of DNA or RNA, for instance a gene of interest placed under the control of adequate promotor and terminator sequences, is coupled to an appropriate cell homing or targetting vector vehicle. When the resulting system is put in the presence of the target cells, the vector-DNA complex will penetrate into said cells, whereby the DNA of interest is internalized in high yields and, whenever desired, can be subsequently expressed.

Naturally, the method is not limited to transfection procedures using only one piece of nucleic acid; co-transfection with two or more sequences is also possible using one or several different homing factors, this being done depending on the case, simultaneously in one step or, successively, in several steps. When effecting a co-transfection with two or more different polynucleotides, for instance, fragments a and b, these fragments can be introduced by linking them separately to the target vectors. Alternatively a and be can be linked together on a same vector unit, the selection of one particular technique depending on the desired results and the operational conditions. In one experiment model developped to demonstrate the feasibility of the concept, the foreign nucleic acids included sequences for regulating the cell development, e.g. using certain eukaryotic cells such as the NIH/3T3 line, said additional sequence was an oncogene of viral or cellular origin which promoted unrestricted cell multiplication in tissue culture, for instance in in-vitro cultures or as tumors after inoculation into warm-blooded animals such as nude mice.

It should be noted that this technique is particularly useful when the foreign gene is designed to express functional products of commercial interest and, consequently, multiplication of the modified host-cells can be performed on a large scale (for instance, industrial production of protein expressed by recombinant DNA dominated by stress inducible promotors).

In the case of in-vivo applications (e.g. treatment of tumors), the foreign nucleic acid can be a heat inducible gene, e.g. a gene coding for a cytotoxic drug and driven by heat-shock controlling sequences. In this case the homing vector is selected for its ability to recognize the malignant cells and promote internalization exclusively therein; then, after effective in-vivo transformation of the tumor cells, heat is applied locally whereby the gene is expressed and the malignant cells are inhibited or killed while leaving the normal cells intact.

## BRIEF DESCRIPTION OF THE FIGURES

Fig 1 is a schematic illustration of the coupling of an oligonucleotide to a cell targetting agent (the epidermal growth factor, EGF is employed here as an example).

Fig 2 is a diagram illustrating the construction of a selected piece of DNA (HS-lys-t/EGF), i.e. a chicken lysosyme gene driven by a human heat-shock promotor and terminated by the SV40 terminator sequence; and the coupling of this selected DNA to EGF.

Fig 3 is a photograph of an electropherogram providing evidence of protein formation under stress in A-431 carcinoma host-cells transformed with the HS-lys-t/EGF coupled system.

Fig 4a-4c are photographs of culture media providing evidence of transformation of "normal" NIH/3T3 cell lines into tumor cells after internalization and expression of an oncogene driven by the EGF homing factor. The black dots represent the "foci" of transformed NIH/3T3 cells.

Fig 5a is a photograph illustrating the formation of tumors in nude mice 3 weeks after inoculation of oncogenically transformed NIH/3T3 cells ("foci") represented in Fig 4. Fig 5b shows a control nude mouse three weeks after inoculation with NIH/3T3 cells treated with EGF only.

Fig 6a represents, under magnification, a culture of a human squamous carcinoma cell line A-431 showing the presence by specific staining of the cell membrane, of EGF receptors. Fig 6b represents, under the same magnification, control cells (WI-38 human embryonic fibroblasts).

## DETAILED DESCRIPTION OF THE INVENTION

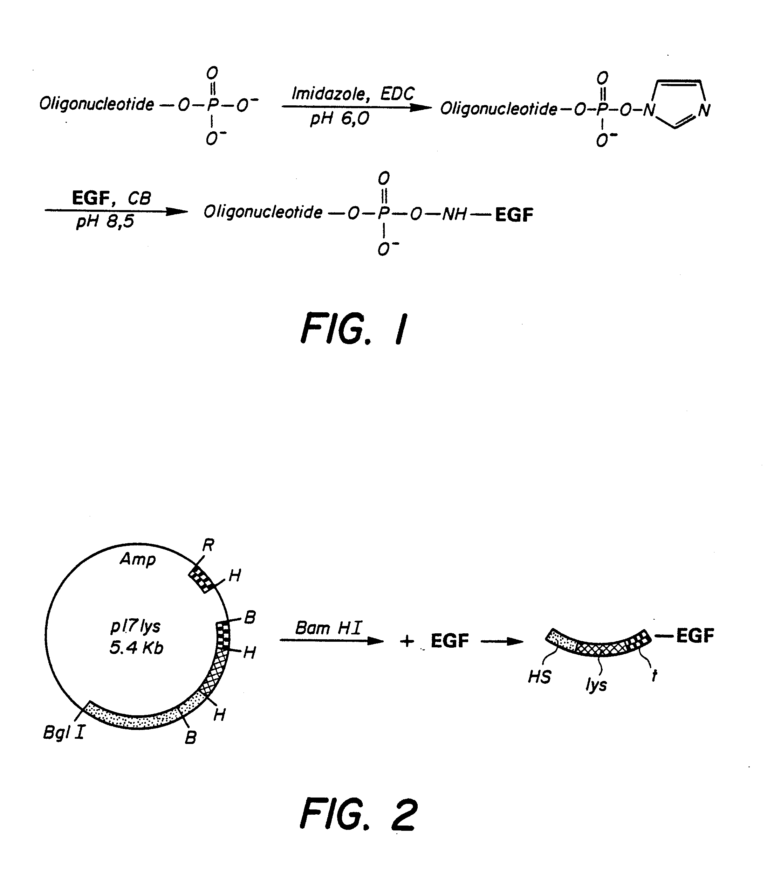

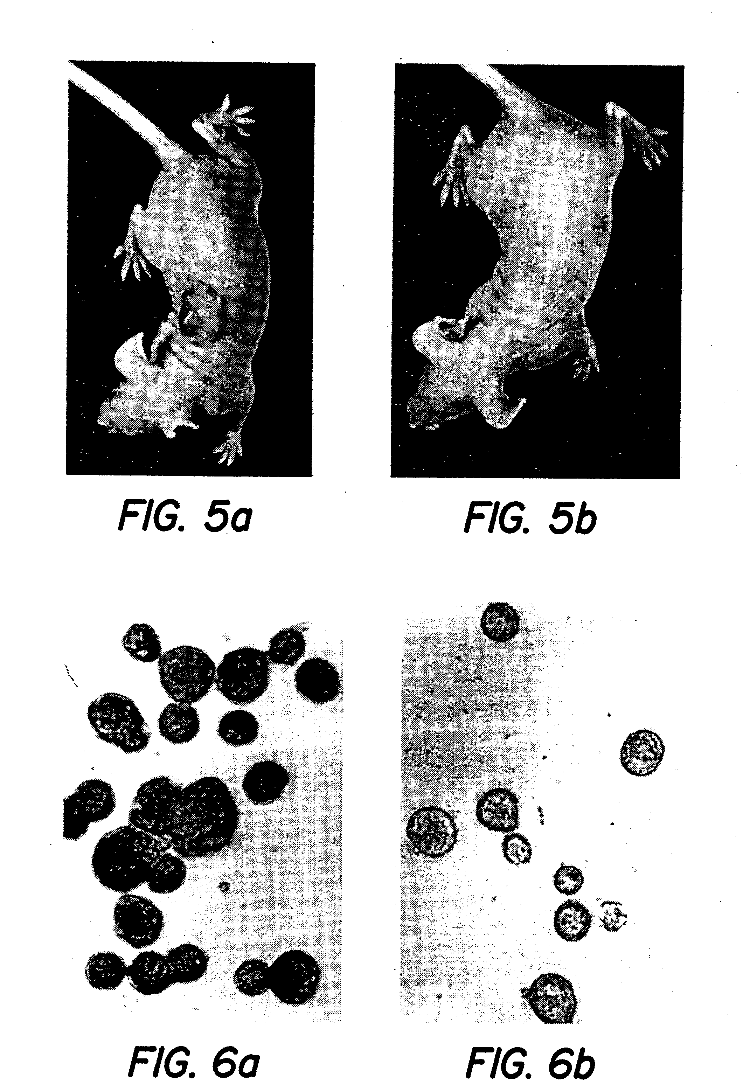

In a first embodiment of the present invention, the epidermal growth factor (EGF) was linked to a 700 bp DNA restriction fragment from the oncogene Ha-ras labelled by kinasing with $^{32}$P dCTP and the resulting complex (I) (see Fig 1) was used to transfect cultures of A-431 cells; and device of these cells (see Fig 6a) was convenient as they carry a large concentration of EGF receptors (see M.D. Waterfield (1982), J. Cell. Bio chem. 20, 149-161).

This coupling was effected according to B.C.F. CHU et.al., (1983), Nucleic Acid Research 11 (18), 6513-6529. This method involves activation of the 5'-phosphate groups of the polynucleotide chain with imidazole at pH 7 and subsequent displacement of this leaving group at pH 8,5 by the terminal aminogroup of EGF (EGF is a polypeptide containing 53 aminoacids, see Savage et al., (1972) J. Biol. Chem. 247, 7609). This is - schematically illustrated in Fig 1.

After 3 hours of incubation, the degree of internalization of the radioactively tagged DNA was measured. It was found that 45% thereof was in the cell cytoplasm, and nucleus, with none in the cellular membrane. A control experiment was performed identically, but in the presence of a 5 fold excess of free EGF for competition. This time the amount of radioactive label in the cytoplasm and nucleus DNA was reduced to 16% providing evidence that internalization of the complex proceeds through the EGF receptors.

In another embodiment, EGF was bound to a piece of DNA schematized by the formula HS-lys-t in which HS is the human heat-shock promoter sequence (Dreano M. et. al., (1986) Gene, in press), lys is the chicken lysozyme gene and t is the SV40 terminator. The scheme illustrating the preparation of this adduct (compound II) is represented in Fig 2.

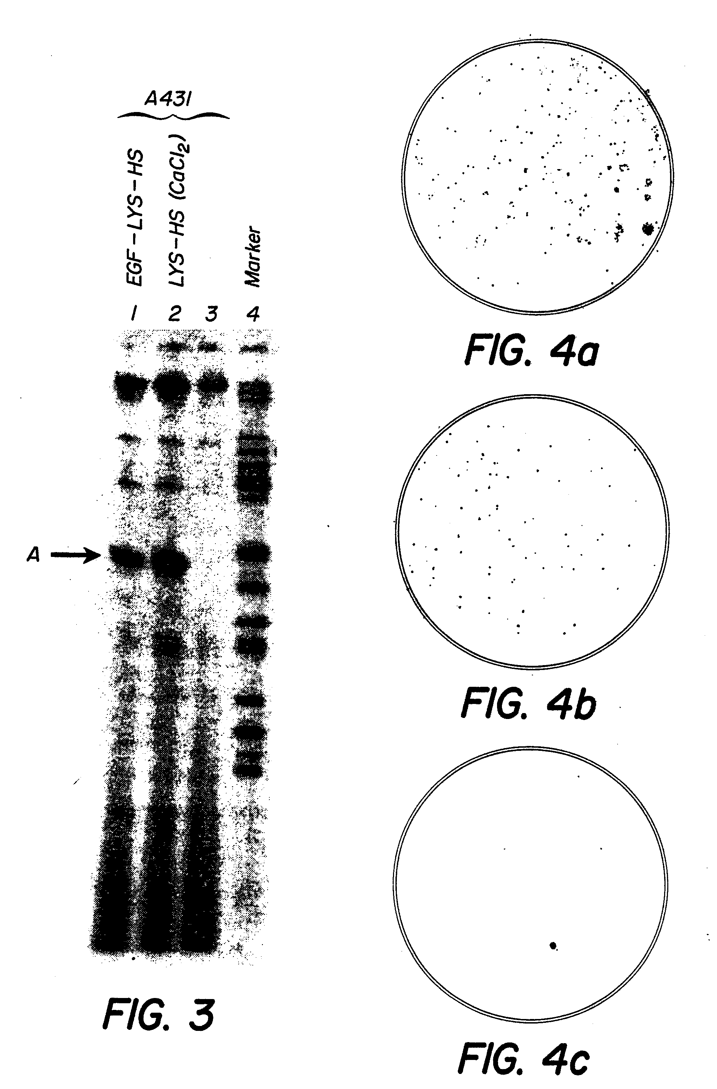

Compound II was introduced into the culture medium of monolayer cultures of A-431 squamous carcinoma cells and, after a period of incubation, the cultures were subjected to a heat stress (4 hours at 42°C), after which the production of lysozyme-specific RNA was ascertained by extraction followed by S-I hybridization. The results appear in the electropherogram of Fig 3.

In Fig 3, lane 1 is the test lane. The lysozyme RNA appears as the dark band (A). Lane 2 (control) is obtained from a culture transfected by the method of the prior art (in the presence of calcium phosphate). Lane 3 illustrates a control experiment in which no compound II was added. Lane 4 is for calibration purposes using markers.

These results conclusively demonstrate the validity of this technique of internalizing DNA and its subsequently induced expression. Naturally, this technique is not limited to structural genes placed under inducible expression, but can be applied to constitutively expressed genes as well.

In a third embodiment the ras oncogene was coupled to EGF; the resulting complex (III) was added to cultures of NIH/3T3 cells and left overnight. After two weeks, the formation of many "foci" was noted, providing evidence of stable cell transformation (i.e. stable incorporation of the "ras" DNA into the "normal" genome). "Foci" are agglomerates of densely packed cells with a refringent phenotype and a tendancy to pile up. The "foci" are the black dots on the photographs of the Petri dishes represented in Fig 4a. Fig 4b illustrates the results of a control experiment in which transfection was carried out with the oncogene (without targetting agent)in the presence of calcium salts according to the prior art. Fig 4c illustrates another control experiment in which only free EGF for adding to the NIH/3T3 cells was used. No observable "foci" were produced this time. The "foci" induced tumor formation when injected into nude mice. Thus, the transformed colonies ("foci") were picked and grown in-vitro until reaching 5 x 10⁶ cells. Then these cells were injected into athymic nude mice. As a control, 5 x 10 ⁶ NIH/3T3 cells treated with EGF (100 μg/ml), were also injected into nude mice. Three weeks after, all the mice inoculated with cells grown from the "foci" developed tumours (Fig 5a), in contrast to the control mice which did not (Fig 5b).

In a second series of controls, injections in nude mice of cells grown from "foci" of NIT/3T3 cells, transfected with the ras oncogene using the calcium chloride method, also induced tumors as expected.

This demonstrates further that the oncogene, introduced into cells according to the invention, is expressed and acts in a stable fashion.

The following examples illustrate the invention in more detail. It should be noted that the operational conditions disclosed are experimental and have not been optimized yet. More specific conditions to be defined later are expected to increase the yields and provide improved results.

## EXAMPLE 1

### COUPLING OF DNA TO EGF

Double-stranded DNA was attached to EGF molecules according to the aforementioned B.C.F. CHU et al. reference (see also EP-A-86 810 347.4).

Genes were excised from plasmids by digestion with endonuclear restriction enzymes, leaving 5′ terminal phosphates. 0,5 to 1 mg of digested plasmid was then run in a 1% agarose gel, and the band corresponding to the gene of interest cut and purified from the gel by electroelution as follows: the gel containing the DNA was introduced into a 5 ml plastic pipet at the end of which was placed siliconized glass fiber and a dialysis bag. The pipet was dipped into TAE buffer (Tris-acetate 0,04M + EDTA 0,001M) and electrophoresed at 150 V for 2 hours. The current was then inversed for two minutes; the DNA recuperated from the dialysis bag, and precipitated twice with ethanol. The first time the DNA was dissolved in water, and the second time, in 20 μl of imidazole buffer 0,1M pH 6.0. The electroelution was controlled by running 1 μl of this solution on a 1% agarose gel.

Formation of a 5′ phosphorimidazolide on the DNA molecules was obtained by saturating the solution with N-ethyl-N′(3-dimethylaminopropylcarbodiimidehydrochloride) (EDC) (FLUKA). The reaction was performed overnight at 4°C with agitation. Aminolysis of imidazolide of the DNA, by the amino terminal extremity of EGF was performed by buffering the solution with 3 volumes of 100 mM citrate buffer (C.B.) pH 8.5, and by adding 4 to 10μg EGF (Sigma), and traces of EGF labelled with ¹²⁵I. (New England Nuclear). Leaving it for 48 hours at 4°C with agitation generated the EGF-DNA conjugate. The free EGF was then separated from the EGF-DNA complex by running the solution over G75 SEPHADEX (column 100 x 2 cm) using a 0,1M ammonium carbonate solution, pH 7.0. The first peak of radioactivity is attributed to the radiolabelled EGF-DNA conjugate. The corresponding fractions were pooled, distributed in aliquots and freeze-dried. Up to 85% of the DNA had an EGF molecule bound to it, as calculated from the DNA concentration (absorbtion at 260 mm) and the number of EGF molecules (amount of radioactivity bound to the DNA).

The EGF-DNA conjugate was kept lyophilyzed in plastic vials at 4°C until rehydration in growth medium for use.

## EXAMPLE 2

### SELECTION OF TEST CELL LINES BASED ON EVIDENCE OF RECEPTOR CONCENTRATION ON THE CELL MEMBRANE USING INDIRECT IMMUNOPEROXYDASE STAINING

Indirect immunoperoxydase staining on A-431 squamous carcinoma and W-138 normal fibroblast human cell lines was performed on trypsinised cells in 35 mm PVC plates. The surface was pretreated with phosphate buffered saline (PBS) pH 7.2, the excess was then removed and the PBS washed cells (10⁵/well in 50 μl/PBS) were added to the plates and centrifuged for 5 minutes at 2000 rpm. 50μl/well of 0,5% glutaraldehyde in cold PBS were then added to the dish and incubated for 15 minutes at room temperature. After two rounds of washes with PBS, the wells were filled with 100 mM glycine in a 0,1% BSA solution and allowed to rest for 30 minutes at room temperature to inhibit the excess of glutaraldehyde. After two PBS washes, indirect immunoperoxydase staining was performed by first denaturing the cells with an ice cold mixture of 99:1 ethanol-acetic acid for 30 minutes at 4°C. During this period, 3 μl of antibody were diluted in 1 ml PBS + 1% foetal calf serum (FCS). The wells were then washed twice with PBS and incubated for 5 minutes with a solution of 20% FCS in PBS. This was then replaced by 200 μl/well of

the antibody solution, and incubated for 30 minutes at room temperature. The wells were then washed twice with PBS, once with PBS + Tween (0,1%) and once again with PBS. 200 μl/well of a 1/400 dilution of swine anti-mouse peroxydase conjugated antibody (DAKO) in PBS-1% FCS was then added and incubated for 30 minutes at room temperature. The conjugate was then washed twice with PBS, once with PBS + 0,1% Tween and twice with distilled water. The in situ coloring was achieved by incubating the cells at room temperature with a solution of 10 ml 0,01M phosphate buffer pH 6.0, 5 μl 35% oxygenated water and 100 μl of 1% orthodianisidin (Merck) in methanol.

Fig 6a represents A-431 tumor cells. The presence of large amounts of EGF receptors therein is indicated by the dark staining. Fig 6b shows the weakly stained W-138 cells containing much lower amounts of EGF receptors which were used as a control.

## EXAMPLE 3

### EGF ~ INTERNALISATION STUDY

EGF was attached to DNA fragments as described in Example 1. The fragment used in this experiment is a 700 base pair probe labelled with $\alpha P^{32}$ ATP by nick-translation using the New England Nuclear System. The internalisation study protocol used here is adapted from a method used by Hillman, G.M. and Schlessinger, J. (Amer. Chem. Soc., 1982, 21, 1667-1672). Briefly, the cell monolayer is incubated with the EGF-DNA mixture, after which the receptors are extracted from the membrane and the cells solubilized for counting the internalized DNA.

Monolayer cell cultures in Petri dishes of 3 cm diameter, were incubated for 3 and a half hours at 37°C with the EGF - DNA complex, labelled with $p^{32}$, in solution A (4 parts DMEM, 1 part of 50mM Tris, 100 mM NaCl and 0,1% BSA adjusted at pH 7.4). The cells were then washed four times with ice-cold PBS + (1mM $CaCl_2$ and 1 mM $MgCl_2$). A 50% trichloroacetic acid solution was added in a proportion of 1/5 to the pooled solution A and PBS + and counted on a scintillation counter. The cell membranes were destabilized by treatment on ice with 200 mM acetic acid and 150 mM NaCl (solution B) for 6 minutes. The solution B was then removed and the cells washed twice with solution B. These pooled solutions were counted for $p^{32}$. This treatment releases the EGF receptors bound to the cell surface. The cells were then completely dissolved in 0,2N NaOH. The radioactivity found then represented the internalized EGF-DNA complex.

To control whether the radiolabelled DNA entered the cell using the internalizing EGF receptor system, competion experiments were set up. The medium was saturated with EGF molecules, thus competitively preventing a large proportion of the EGF - DNA complex from interacting with the EGF receptor. 2μg of free EGF was added to the medium containing the radiolabelled EGF - DNA complex, and the internalized radioactivity was counted after 3 and a half hours of incubation at 37°C.

In these experiments, as much as 45% of the radiolabelled EGF - DNA complex added to the medium was found in the intracellular compartment after 3 and a half hours of incubation. Under the same conditions, when the medium was saturated with free unlabelled EGF, the internalized EGF - DNA complex was reduced to 1/3, displaying only 16% of internalized product.

These results taken together provide evidence that DNA can be carried into the cell using the EGF receptor system.

## EXAMPLE 4

### GENE CONSTRUCT CONJUGATED TO EGF ENTERS THE CELL AND IS TRANSIENTLY EXPRESSED

A human heat inducible promoter, fused to a chicken lysozyme gene has been used as a model in a transient expression system. Such a construct, termed p17lys, has been demonstrated to function in monkey COS cells and in xenopus oocytes (Dreano M. et al., 1986, Gene in press). p17lys was condensed with EGF (as described in example 1) and the EGF - p17lys complex was mixed in complete growth medium and added to A-431 monolayer cell cultures as follows.

The cells were first washed twice with DMEM without serum and left to incubate for 4 hours in medium without serum. The EGF - p17lys complex was then added to 1 ml of medium without serum. After 3 hours incubation at 37°C, 9 ml of DMEM, 10% FCS was added. After an overnight incubation, the cells were heat shocked at 42°C for 4 hours and lysed for extraction of the RNA.

RNA extraction was performed by treating PBS washed cells with a solution containing 10mM Tris pH 7.5, 1mM $MgCl_2$, 10mM NaCl, 1% SDS and 1 mg/ml fresh proteinase K. The cells were left in this solution on ice for 1 hour after which NaCl was added to make 0,3 M. The cell debris were scraped off and centrifuged 10 minutes at 11000 r.p.m. in an airfuge at 4°C. The supernatant was then removed and precipitated in ethanol for 5 minutes in a dry ice-ethanol bath. After a 15 minutes centrifugation, the supernatant was discarded and the pellet redissolved in 20 $\mu$l of 10 mM PIPES, pH 6.9, 0,4M NaCl, 1mM EDTA, 50% formamide. The hybridization with a 400 bp. fragment of the lysozyme gene construct was performed according to H.G. Stummenberg and M.L. Brinstiel (P.N.A.S., USA, 1982, Vol 79, pp 6201-6204). Aliquots of about 30 $\mu$g of total RNA were used for each assay. The SI protection assay was carried out according to Weaver, R.F. and Weissmann, C., (1979, Nucl. Acids Res., 6, 1175-1193 except that DNA fragments were not heated in pure formamide but resuspended and denatured directly in hybridization buffer (Dierks et al., 1981, Cell, 32, 695-706). Nuclease SI was from PL/Pharmacia. After digestion, excess RNA was eliminated in 0,1N NaOH at 100°C for 3 min. The protected fragments were analysed on 7M urea, 8% acrylamide gels (0,4 mm). End-labeled HaeIII fragments of pBR322 were used as molecular weight markers.

## EXAMPLE 5

### A GENE CONSTRUCT LINKED TO EGF ENTERS THE CELL AND BRINGS ABOUT STABLE TRANSFORMATION

A well documented system is the transformation of NIH/3T3 mouse fibroblasts by ras oncogenes using calcium chloride transfection technique (Graham, F.L. and Van der Eb, A.J., 1973, Virology, 52, 456-467 Wigler, M. et al., 1979, PNAS 76, 1373-1376). The transformed cells form foci of refringent cells that have lost contact inhibition and can grow in athymic mice.

We have fused the human ras oncogene to an EGF molecule as described in example 1, and have tested this complex on NIH/3T3 cells. Confluent monolayers of NIH/3T3 cells were washed twice with DMEM without serum, and were incubated in this medium for 4 hours. After this, the EGF - ras complex was added to the medium and allowed to incubate for 3 hours at 37°C. Complete DMEM medium with 10% FCS was then added to the Petri dish, in a proportion of 10/1 (v/v), and left overnight at 37°C. The next day the cells were split in 1/20, 1/40 and 1/80 dilutions, and left growing for 17 days after which the foci were scored.

This experimental procedure indicates that, using EGF, the ras oncogene could be internalized into the cell, brought to the nucleus and could be expressed, thereby transforming NIH/3T3 in a stable fashion. The results obtained show that the EGF-ras complex transforms with a greater efficiency than transfections using the calcium phosphate technique. About 5 times more foci were obtained in the 1/40 cell dilution using the EGF-ras complex (Figure 4).

EGF-ras 438 foci
CaCl$_2$,ras 82 foci
Control 8 foci

Foci were gathered, expanded to cultures of 5-10$^6$ cells and injected into athymic nude mice. The EGF-ras colonies as well as the CaCl$_2$ transfected ras gene form tumours within 3 weeks, whereas control NIH/3T3 cells untreated or just treated with an excess of EGF for 24 hours do not form any tumours in nude mice.

In other experiments designed to demonstrate the operability of in vivo gene therapy, targeted complexes composed of a gene coding for a cytotoxic protein (e.g. the ricin diphteria or pseudomonas toxin gene) linked to a homing vector such as EGF or any other tumour cell specific marker are injected into cancerous animals, either locally or in the blood stream. Then, if the promotor selected makes the gene heat inducible, local heating the subject induces the expression of the toxin only in the region of the tumour, whereby the latter is destroyed, without harmful effects to healthy tissues. Otherwise, similarly built targetted complexes but comprising other promoters, e.g. for controlling expression by chemical induction, can be used similarly.

Evidently the invention is not limited to the above embodiments but it can be used in many other applications involving internalization of DNA in vitro and in vivo.

Among such applications, the following may be cited :

1. Industrial production of proteins of interest by the inducible or continuous expression of genes efficiently internalized via cytospecific gene vectors according to the present invention. Therefore, large quantities of a protein of interest can be produced by cells grown in fermentors or by tumour cells carried by laboratory animals. This invention allows one to bypass the long and troublesome steps of establishing cell lines producting a selected protein. It may also be possible to internalize RNA complexes which could be directly translated to proteins.

2. Cytospecific drugs consisting of an internalizable molecule, linked to a nucleic acid expressing a protein which can have various types of activities, for example therapeutic, prophilactic, antivirial, cytotoxic, or may have an effect on the regulatory mechanism of the cell. The gene can also complement a genetic defect or serve as a marker. Such drugs can be used in vivo or in vitro.

3. The present method of introducing DNA or RNA in the cell gives a whole new dimension to gene therapy because of the possibility of working without using any toxic agents and because it allows efficient targetting of nucleic acids to specific cell populations. Using this internalization technique, modulation of gene expression (e.g. repression) using anti-sense RNA or DNA becomes a very powerful and versatile method.

**Claims**

1. Method for transfecting cells, i.e. for introducing into appropriate host-cells one or more selected pieces of foreign polynucleotides (nucleic acids such as DNA or RNA), which comprises the steps of :
(1) making a coupled complex by chemically coupling said piece(s) of nucleic acid to a ligand having high affinity for predefined recepter sites of said host-cells, said ligand being capable of acting as a cell targetting agent toward said cells and as a cell internalization vector;
(2) exposing said host-cells to the coupled complex resulting from step (1), these cells being selected for the presence thereon of a significant quantity of said receptors, for a time sufficient to achieve effective penetration into the cells and internalization of said coupled nucleic acid.

2. The method of claim 1, wherein the polynucleotide is chosen from RNA, single stranded DNA and duplex DNA and derivatives thereof.

3. The method of claim 2, wherein the polynucleotide is a sequence containing a functional gene of interest and untranslated sequences.

4. The method of claim 3, wherein the gene codes for identifiable product expressed under control of the untranslated sequences.

5. The method of claim 3, wherein the gene codes for anti-sense RNA for blocking viral RNA or cellular RNA functions.

6. The method of claim 3, wherein the gene codes for a biologically active product selected from cytotoxins, hormones, enzymes, lectins, antibodies, antisense RNA and DNA, etc, under the driving action of a stress inducible promotor.

7. The method of claim 6, wherein the promotor is a heat-shock control sequence of eukaryotic origin.

8. The method of claim 1, wherein said cell receptor is selected among cell growth factors (e.g. EGF), synthetic and natural derivatives thereof, antibodies and cell specific markers.

9. The method of claim 3, comprising the additional steps of :
(3) inducing expression of said gene of interest into useful products and isolating said products.

10. The method of claim 2, wherein the polynucleotide comprises an inducible gene coding for a product of interest and the host cell is eukaryotic and unrestricted multiplications, the method comprising the following additional steps of:
(3) inoculating the transformed cell lines into immunodeficient warm-blooded animals which develop tumors in response to inoculation and allowing said tumors to grow to $10^6$-$10^{10}$ cells;
(4) dissociating said tumors into individual cells, cultivating said dissociated cells in culture media;
(5) subjecting said cultures to stress whereby said gene is expressed; and
(6) collecting and purifying said product of interest.

11. The method of claim 6 in which the gene codes for a cytotoxin, comprising effecting step (2) by injecting said coupled complex into the blood stream or a living tissue containing normal cells and tumoral cells bearing said receptor sites, whereby said coupled complex is selectively internalized in the tumoral cells, then
(3) stressing local said tissue to induce the production of the cytotoxin, whereby the tumoral cells are inhibited or destroyed while leaving the normal cells unharmed.

12. The method of claim 2 involving co-transfection with at least two independent polynucleotides, said nucleotides being coupled to one or to two different ligands.

13. As a new composition of matter, a cell transforming vector consisting of a nucleic acid sequence chemically coupled to a ligand with specific affinity toward a receptor of the cellular membrane, this receptor promoting internalization of said nucleic acid sequence in high yield.

$$\text{Oligonucleotide} - O - \overset{\overset{O}{\|}}{\underset{\underset{O^-}{|}}{P}} - O^- \quad \xrightarrow[\text{pH 6,0}]{\text{Imidazole, EDC}} \quad \text{Oligonucleotide} - O - \overset{\overset{O}{\|}}{\underset{\underset{O^-}{|}}{P}} - O - N \underset{N}{\bigcirc}$$

$$\xrightarrow[\text{pH 8,5}]{\textbf{EGF, CB}} \quad \text{Oligonucleotide} - O - \overset{\overset{O}{\|}}{\underset{\underset{O^-}{|}}{P}} - O - NH - \textbf{EGF}$$

## FIG. 1

## FIG. 2

FIG. 3

FIG. 4a

FIG. 4b

FIG. 4c

FIG. 5a

FIG. 5b

FIG. 6a

FIG. 6b

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | NUCLEIC ACIDS RESEARCH, vol. 11, no. 3, 1983, pages 654-669, Oxford, GB; S.-Y. CHENG et al.: "A versatile method for the coupling of protein to DNA: synthesis of alpha2-macroglobulin-DNA conjugates" * Abstract; discussion * | 1 | C 12 N 15/00 |
| D,Y | NUCLEIC ACIDS RESEARCH, vol. 11, no. 18, 1983, pages 6513-6529, Oxford, GB; B.C.F. CHU et al.: "Derivatization of unprotected polynucleotides" * Abstract; page 6528, lines 10-21 * | 1 | |
| A | NUCLEIC ACIDS RESEARCH, vol. 12, no. 14, 1984, pages 5707-5717, Oxford, GB; M.A. LOPATA et al.: "High level transient expression of a chloramphenicol acetyl transferase gene by DEAE-dextran mediated DNA transfection coupled with a dimethyl sulfoxide or glycerol shock treatment" * Abstract; introduction; discussion * | 1 | **TECHNICAL FIELDS SEARCHED (Int. Cl.4)** <br><br> C 12 N |

-/-

The present search report has been drawn up for all claims

| Place of search | Date of completion of the search | Examiner |
|---|---|---|
| THE HAGUE | 27-08-1987 | YEATS S.M. |

European Patent Office

**EUROPEAN SEARCH REPORT**

| | DOCUMENTS CONSIDERED TO BE RELEVANT | | Page 2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | PROCEEDINGS OF THE NATIONAL ACADEMY OF SCIENCES OF THE USA, vol. 76, no. 3, March 1979, pages 1373-1376, Washington, US; M. WIGLER et al.: "DNA-mediated transfer of the adenine phosphoribosyltransferase locus into mammalian cells" <br> * Abstract; page 1373, column 2, line 37 - page 1374, column 2, line 23; table 1; discussion * <br><br> --- | 1 | |
| T | THE JOURNAL OF BIOLOGICAL CHEMISTRY, vol. 262, no. 10, 5th April 1987, pages 4429-4432, The American Society of Biological Chemists, Inc., US; G.Y. WU et al.: "Receptor-mediated in Vitro gene transformation by a soluble DNA carrier system" <br> * Abstract; introduction; results; discussion * <br><br> ----- | 1 | TECHNICAL FIELDS SEARCHED (Int. Cl.4) |

The present search report has been drawn up for all claims

| Place of search | Date of completion of the search | Examiner |
|---|---|---|
| THE HAGUE | 27-08-1987 | YEATS S.M. |